# EUROPEAN PATENT SPECIFICATION

(11) **EP 3 098 302 B1**
(45) Date of publication and mention of the grant of the patent: **17.05.2017**
(21) Application number: 15169066.6
(22) Date of filing: 25.05.2015
(51) Int. Cl.: C12N 1/20, A23K 10/18, A23L 29/00, C12R 1/25

(54) **LACTOBACILLUS PLANTARUM STRAIN P 1462 AND PRODUCTS CONTAINING THE STRAIN**
LACTOBACILLUS PLANTARUM STAMM P 1462 UND PRODUKTE, DIE DEN STAMM ENTHALTEN
SOUCHE LACTOBACILLUS PLANTARUM P 1462 ET PRODUITS CONTENANT CETTE SOUCHE

(43) Date of publication of application: 30.11.2016
(73) Proprietor: Latvijas Universitate, 1586 Riga (LV)
(72) Inventor: Semjonovs, Pavels, 1024 Riga (LV); Upite, Dagnija, 5000 Ogre (LV); Auzina, Lilija, 1055 Riga (LV); Patetko, Arturs, 1058 Riga (LV); Petranis, Arnis, 1009 Riga (LV); Denina, Ilze, 1010 Riga (LV); Upitis, Andris, 5000 Ogre (LV)
(74) Representative: Fortuna, Aleksandra

(56) References cited:
- ALEXANDER GELMAN ET AL: "Evaluation of lactic acid bacteria, isolated from lightly preserved fish products, as starter cultures for new fish-based food products", INNOVATIVE FOOD SCIENCE AND EMERGING TECHNOLOGIES, vol. 1, no. 3, 1 September 2000 (2000-09-01), pages 219-226, XP55226506, NL ISSN: 1466-8564, DOI: 10.1016/S1466-8564(00)00023-0
- XUEFENG ZENG ET AL: "Technological properties of Lactobacillus plantarum strains isolated from Chinese traditional low salt fermented whole fish", FOOD CONTROL., vol. 40, 1 June 2014 (2014-06-01), pages 351-358, XP55226688, GB ISSN: 0956-7135, DOI: 10.1016/j.foodcont.2013.11.048
- NIE XIAOHUA ET AL: "Proteolytic characterisation in grass carp sausage inoculated with Lactobacillus plantarum and Pediococcus pentosaceus", FOOD CHEMISTRY, vol. 145, 15 February 2014 (2014-02-15), pages 840-844, XP028741884, ISSN: 0308-8146, DOI: 10.1016/J.FOODCHEM.2013.08.096
- L.-J Yin ET AL: "JFS: Food Microbiology and Safety New Technology for Producing Paste-like Fish Products using Lactic Acid Bacteria Fermentation", , 1 January 2002 (2002-01-01), XP55226742, Retrieved from the Internet: URL:http://onlinelibrary.wiley.com/store/1 0.1111/j.1365-2621.2002.tb08867.x/asset/j. 1365-2621.2002.tb08867.x.pdf?v=1&t=igrsd59 x&s=0ce8e99037d6a61d21571d96be74f6cdeedc6c cf [retrieved on 2015-11-09]
- XUEFENG ZENG ET AL: "Biochemical and Sensory Characteristics of Whole Carp Inoculated With Autochthonous Starter Cultures", JOURNAL OF AQUATIC FOOD PRODUCT TECHNOLOGY, vol. 24, no. 1, 2 January 2015 (2015-01-02), pages 52-67, XP55226746, US ISSN: 1049-8850, DOI: 10.1080/10498850.2012.754535

## Description

### Technical Field

The invention relates to food microbiology, food technology and biotechnology sector and is directed to production of fermented products and concentrates from fish and marine origin raw materials.

### Background Art

More and more studies in recent years have convincingly demonstrated the importance of balanced food for human health maintenance. Consequently, consumers usually prefer food without chemical preservatives and other chemical additives, besides an important role also has microbiological safety. A crucial criterion for the quality of food is its safety during storage and transportation. One way to ensure this, is food processing technology known since ancient times - production of fermented food products, using GRAS (generally recognized as safe) microorganisms' starter cultures. Popularity of such foods is still growing, because also its production costs are relatively low [1]. Raw materials can be both of vegetable and animal origin. It is known, that fish and marine origin raw materials can also be fermented with the appropriate microbial starter cultures. It has a great variety of original ancient traditions, especially in Asia countries. Recently extensive research has been conducted not only with traditional raw materials, but also with non-standard ones, including fish processing by-products. Fish non-standard production and industrial processing by-products are large untapped potential in terms of volume and potentially acquired added value. Every year up to 30 million tons of fish processing by-products wasted worldwide [2]. Although some part of fish by-products are used as raw material in the production of animal feed, the residual amount is still disproportionately large. By using biotechnological approaches these products have a great potential to be used as raw materials in manufacturing of high-quality products - food, pharmaceuticals, cosmetics, thus providing a multiple increase of added value. Fish products or low-value fish species can be used as a protein source with high biological value, a source of unsaturated, essential fatty acids, amino acids, antioxidants and vitamins. It is known that fish and fish products also contain omega-3 polyunsaturated fatty acids, which reduce the risk of acquiring coronary cardiovascular disease and diabetes, promotes recovery in cases of different types of arthritis, of neurological disorders, sight and reproductive system dysfunction.

If these natural biological materials are processed by using environmentally friendly processing technologies, the products obtained acquire higher long-term added value. One of such technologies is the microbial fermentation with starter cultures. In order to intensify the process, there is carried out intensive research in the world on selection of starter cultures being suitable for certain raw materials, including ones of fish origin. The most commonly used starter cultures and the combination thereof contain lactic acid bacteria [3], [4], [5] e. g., *Lactobacillus acidophillus, L. plantarum,* due to their ability to give the final product a pleasant sensory properties: good taste, aroma, texture features, relatively longer shelf-life, and the antimicrobial effect [6]. In particular Lactobacillus plantarum strains have been used for the preparation of fish products, such as minced tuna or fish surimi (Gelman A. et al., Innovative food science and emerging technologies (2000), vol. 1, no. 3: 219-226 and Zeng X. et al., Food control (2014), vol. 145: 351-358). It is known that unwanted microflora, which poses a serious health safety risks in the food production process and finished products, cannot survive in an acidic environment (below pH 4). It has been proved that the *Escherichia coli* enterotoxic strains during fermentation under acidic conditions are inactivated [1]. Bacteriocins of lactic acid bacteria are considered safe natural inhibitors, the use of which in food production represents a new approach for inactivating of pathogenic microflora in food products. So nisin was the first antimicrobial polypeptide isolated from lactic acid bacteria [7]. Today nisin preparations as natural preservatives are allowed for use in at least 48 countries - in production of cheese, meat products and canned products. It should be noted that lactic acid bacteria fermented food can often be qualified as functional food, because of possible presence of certain probiotic strains. In the result of fermentation with lactic acid bacteria, fish products obtain additional qualitative characteristics: they are enriched with probiotics, due to proteolytic and other enzymes activity they become more easily digestible, fermented products are microbiologically safer - because of lower pH, promoted by the organic acids and synthesized bacteriocins - significantly increases their sanitary safety and shelf-life, increases and appears characteristic organoleptic properties. Dehydration of the fermented mass by the way of its lyophilization, allows to use the obtained preparations in the form of dry food supplements, because the product is easy to transport, it is stable and could be stored for a long time.

### Disclosure of the Invention

A new lactic acid bacteria strain of *Lactobacillus plantarum* P 1462 and products fermented by it are proposed, such as products of marine origin and fish processing products and their dehydrated concentrates, i.a. as food or feed supplements.

Lactic acid bacteria *Lactobacillus plantarum* strain P 1462 has been isolated from the Baltic herring (*Clupea harengus membras*)*.* The strain has been deposited in the Latvian Culture Collection of Microorganisms under the accession number P 1462.

Cells of *Lactobacillus plantarum* strain P 1462 are rod-shaped (1.0 µm x 2.0 -20.0 µm), even or odd, non-motile, they do not form spores, Gram - positive, facultative anaerobic, no nitrate reduction observed, no H₂S synthesizing. Catalase, and cytochrome oxidase reaction is negative. On agarized MRS medium (Oxoid, CM361) colonies are shiny, white, smooth, optimum growth temperature +35 - +37 C, growing temperatures range +12 - +40 °C.

2.0-5.0% of P 1462 inoculum with the concentration of 10⁶ - 10⁸ cfu/ml can be added to the fermentable fish or seafood substrate.

### Brief Description of Drawings

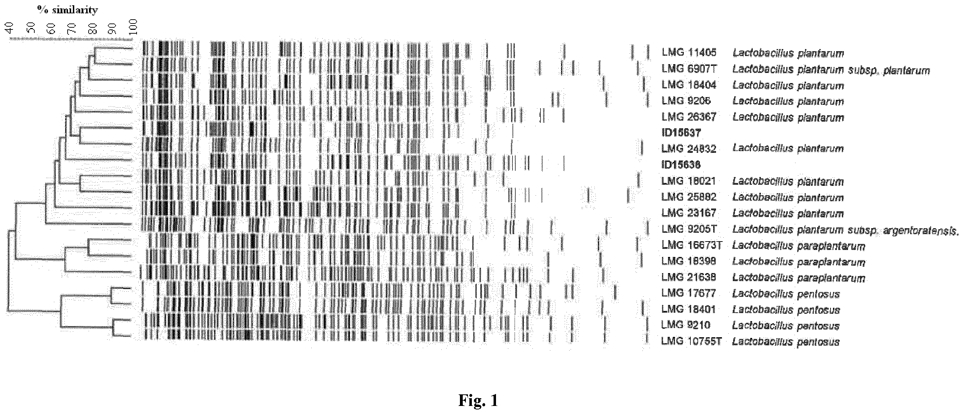

Fig. 1 is the homology and phytogenetic tree of *Lactobacillus plantarum,* wherein the proposed strain P 1462 is shown as ID 15637;

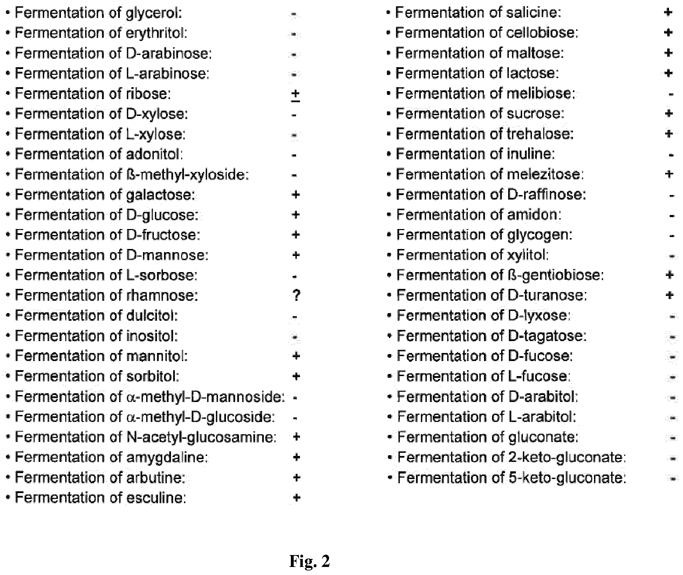

Fig. 2 shows *Lactobacillus plantarum* P 1462 biochemical properties using API 50 CHL identification system (the results obtained at 37 °C, 48 h), where "+" means positive, "-"-negative "?" - the reaction is not clear, "±" - the reaction is weakly positive.

### Examples of the Implementation of the Invention

The following examples illustrate some application variants of *Lactobacillus plantarum* P 1462.

### Example 1

Baltic herring is washed and minced; 2% of glucose, 1% salt (NaCl), distilled water 10% are added to the mass. The mass with these additives is sterilized for 20 min. in scavenging steam, further 10 min. at 0.5 bar. After cooling, starter microorganisms (pure cultures): *Lactobacillus plantarum* P 1462, *Lactobacillus plantarum* B51 and *Lactobacillus paraplantarum* B85 are added to samples. The inoculum is added at a concentration of 2% (ODλ₅₅₀ = 0.770, overnight culture). Before fermentation the pH value of the mass = 6.5±0.2.

**Table 1. Fermentation of herring mince with Lactobacillus plantarum strains P 1462, B51 and Lactobacillus paraplantarum B85, where 2% of glucose, 1% of NaCl, and 10% of distilled water are added (48 h, 35°C).**

| Inoculum | pH | Cell-count, cfu/1g | Lactic acid, g/l | Acetic acid, g/l | Formic acid g/l | Propionic acid g/l |
|---|---|---|---|---|---|---|
| P 1462 | 3.7 | 0.2x10⁹ | 33.462 | 1.781 | 0.200 | 0.903 |
| B85 | 5.02 | 0.4x10⁸ | 15.969 | 3.571 | 0.205 | 0.520 |
| B51 | 4.8 | 0.5x10⁸ | 21.521 | 0.716 | 0.203 | 0.656 |

After fermentation for 48 hours at a 35°C under the same conditions the better result has a sample with starter *Lactobacillus plantarum* (P 1462), i.e., pH is significantly lower, the viable cell count reaches 0.2x10⁹ cfu/g, significantly exceeding characteristics of fermentations obtained with other strains, concentration of lactic acid is higher (33.5 g/l) than in samples with starter cultures of *Lactobacillus paraplantarum* (B85) and *Lactobacillus plantarum* (B51).

### Example 2

Baltic herring is washed and minced; 2% of sucrose, 2% of salt (NaCl), 10% of distilled water are added to the mass and sterilized for 20 min. in scavenging steam, following by 10 min. at 0.5 bar). After cooling, inoculum of *Lactobacillus plantarum* (P 1462), *Lactobacillus* sp. (B87) and *Lactobacillus sp.* (B93) has been added to samples. The inoculum is added at the concentration of 2 % (ODλ₅₅₀ = 0.770, overnight culture). Before the fermentation pH value of the mass was 6.5±0.2.

**Table 2. Fermentation of herring mince with Lactobacillus plantarum P 1462 and Lactobacillus sp. strains B87 and B93, where 2% of sucrose, 2% of NaCl, and 10% of distilled water are added (48 h, 35°C).**

| Inoculum | pH | Cell-count, cfu/1g | Lactic acid, g/l | Acetic acid, g/l | Formic acid, g/l | Propionic acid g/l |
|---|---|---|---|---|---|---|
| P 1462 | 2.96 | 4x10⁹ | 32.171 | 1.654 | 0.197 | 0.832 |
| B87 | 5.1 | 4.7x10⁷ | 14.714 | 2.737 | 0.282 | 0.483 |
| B93 | 4.9 | 1x10⁷ | 16.453 | 2.053 | - | 0.591 |

After fermentation for 48 h at 35°C under the same conditions the better result has a sample with starter *Lactobacillus plantarum* (P 1462), i.e., pH is lower, the cell count (cfu/g) is significantly higher, reaching 4.0 x10⁹ cfu/g, than that for other cultures, lactic acid concentration is significantly higher (32,171 g/l), than in samples with starter cultures *Lactobacillus sp.* (B87) and *Lactobacillus sp.* (B93).

Example 2 differs from the Example 1 with different added carbon source (sucrose 2%) and increased salt concentration (2%). Also in these circumstances it appears that *Lactobacillus plantarum* starter P 1462 provides more active fermentations process, which testifies its good salt tolerance characteristics and the presence of sucrose hydrolysing enzymes. Using s P 1462 strain starter culture, fermentation rates after 48 hours are considerably higher: i.e., pH is significantly lower, which can be explained by significantly more active synthesis of lactic acid, the number of lactic acid bacteria cfu/g is significantly higher than that of other cultures, reaching 4.0 x10⁹ cfu/g.

### Example 3

Salmon fillets are washed, blendered (homogenised); 2% of sucrose, 2% of salt, 10% of distilled water are added to the mass and sterilized for 20 min in scavenging steam, after that 10 min at 0.5 bar. After cooling, inoculum of *Lactobacillus plantarum* (P 1462), *Lactobacillus sp.* (B87) and *Lactobacillus sp.* (B93) is added. The inoculum is added at the concentration of 2% (ODλ₅₅₀ = 0,770, overnight culture). Before fermentation the pH value of the mince is 6.5±0.2.

**Table 3. Fermentation of salmon mince with Lactobacillus plantarum P 1462 and Lactobacillus sp. strains B87 and B93, where 2% of sucrose, 2% of NaCl, and 10% of distilled water (48 h, 35°C) is added.**

| Inoculum | pH | Cell-count, cfu/1g | Lactic acid, g/l | Acetic acid, g/l | Formic acid, g/l | Propionic acid, g/l |
|---|---|---|---|---|---|---|
| P 1462 | 4.6 | 4x10⁸ | 28.12 | 2.178 | 0.176 | 0.405 |
| B87 | 4.75 | 0.2x10⁷ | 14.61 | 3.164 | 0.318 | 0.891 |
| B93 | 5.01 | 3x10⁶ | 10.47 | 5.153 | 0.288 | 0.602 |

After fermentation for 48 h at 35°C under the same conditions the better result has the sample with *Lactobacillus plantarum* (P 1462) starter culture, i.e., pH is lower, cell count is significantly higher (4x10⁸ cfu/g), lactic acid concentration is higher (28.12 g/l) than in samples with starter cultures *Lactobacillus sp.* (B87) and *Lactobacillus sp.* (B93).

Considering that there are objective differences between properties of salmon and Baltic herring minces - i.e., in content, consistency, texture, etc., fermentation with starter *Lactobacillus plantarum* P 1462 shows that fermentation parameters both in Examples 1 and 2 are better than of other cultures: pH value is lower, cell count of lactic acid bacteria per 1g of weight is higher, also concentration of lactic acid (28.12 g/l) is higher. After freeze-drying powdered fermented salmon concentrate with a gentle fish flavour can be obtained.

### Example 4

Baltic herring mince and inoculum had been prepared as described in Example 1. Salt (NaCl) at the concentration of 1% and glucose at the concentration of 2% are added to the herring mince. After supplementation the mince has been sterilized as described in Examples 1-3. Fermentation has been carried out as described in Examples 1-3.

The obtained fermented herring mass samples were dehydrated by the way of lyophilisation (freeze-drying). The fermented samples were filled into the vessels of laboratory freeze -drier *Vir Tis Bench Top 6K.* These vessels were placed in the refrigerator freezer at -20°C and kept there for 24 hours. Further the frozen product was placed in lyophilization chamber, where it was frozen up to -40°C and kept at this temperature for 3 hours. When the vacuum pump was started, sublimation of ice present in the product started. During this time the pressure maintained in the operating chamber was not higher than 10 Pa (0.075 mTorr). In those conditions the product was held for 10 hours. Then heat was supplied to the product for 10 hours with the heating rate, so that the product's temperature did not exceed -20°C. Further supply of the heat was increased, not allowing the product to reach more than +35°C. Such a regime was maintained for 10 hours.

**Table 4. Lyophilization of the fermented Baltic herring mass (starter cultures Lactobacillus plantarum strains P 1462, B51 and Lactobacillus paraplantarum strain B85) for obtaining dry preparation**

| Inoculum used for fermentation | Lactic acid bacteria cell-count, cfu/1g of dry matter | | Dry matter, % | |
|---|---|---|---|---|
| | Before lyophilization (in the fermented mass) | After lyophilization | Before lyophilization | After lyophilization |
| B51 | 0.4x10⁸ | 0.3x10⁷ | 21.85 | 92.1 |
| P 1462 | 0.9x10⁸ | 0.72x10⁸ | 22.62 | 92.3 |
| B85 | 1.8x10⁸ | 0.4x10⁷ | 22.12 | 92.3 |

After the lyophilization process the cell count of lactic acid bacteria in the samples was tested in 1g of dry preparation (calculated for the dry matter content of the sample). After examination of the survival of lactic acid bacteria during the lyophilization, it was found out, that *Lactobacillus plantarum* P 1462 shows significantly higher endurance in the lyophilization process, thus ensuring a higher lactic acid bacterial survival in the preparation of fermented Baltic herring dry mince, as compared to *Lactobacillus paraplantarum* B51 and *Lactobacillus plantarum* B85 strains.

It is shown that the isolated and identified lactic acid bacteria *Lactobacillus plantarum* strain P 1462 can be recognized as more efficient for fish and fish-origin raw materials fermentation and subsequent dehydration by the way of freeze-drying. As a result of use of said strain the dehydrated, powdered microbiologically safe fermented fish and marine origin products can be obtained, which, depending on the raw material composition and origin, can be used as a food or feed supplements.

### Sources of Information

[1] Peter Sahlin. Fermentation as a Method of Food Processing. Lund University Department of Applied Nutrition and Food Chemistry, 1999.
[2] [Dragnes, B.T. and Elvevoll, E.O. (2003). Documantation and novel functions of marine by-products, Taft poster, Reykjavik, Iceland, June 11-14.
[3] R.Kolfi-Nevry, T.S.T.Ouina, M.Koussemon and K.Brou. Chemical Composition and Lactic Microflora of Adjuevan, A Tradicional Ivorian Fermented Fish Condiment. Pakistan Journal of Nutrition 10 (4) (2014), pp. 332-337.
[4] Corsetti, A., Settanni, L., Van Sinderen, D. 2004. Characterization of bacteriocin-like inhibitory substances (BLIS) from sourdough lactic acid bacteria and evaluation of their in vitro and in situ activity. Journal of Applied Microbiology 96 (3), 521-534.
[5] Xiaohua Nie, Shengli Lin, Qilin Zhang. Proteolytic characterisation in grass carp dryge inoculated with Lactobacillus plantarum and Pediococcus pentosaceus. Food Chemistry 145 (2014); pp.840-845.
[6] Schnurer J. And Magnusson J: Antifungal lactic acid bacteria biopreservatives. Trends in Food Science and Technology. 16. 70-80 (2005).
[7] Roger, L.A. 1928. The inhibity effect of Streptococcus lactis on Lactobacillus bulgaricus.

## Claims

1. The lactic acid bacteria *Lactobacillus plantarum* strain, deposited in Latvian Culture Collection of Microorganisms under number P 1462.

2. Products, containing *Lactobacillus plantarum* strain P 1462 as per claim 1.

3. Use of *Lactobacillus plantarum* strain P 1462 for production of fermented fish and marine origin products and their processing by-products.

4. Use of *Lactobacillus plantarum* strain P 1462 for production of fermented and dehydrated fish and marine origin products and their processing by-products.

## Patentansprüche

1. Der Milchsäure-Bakterienstamm *Lactobacillus plantarum* wurde bei der Lettischen Kultursammlung von Mikroorganismen unter der Nummer P 1462 deponiert.

2. Produkte, die gemäß Anspruch 1 den Stamm *Lactobacillus plantarum* P 1462 enthalten.

3. Verwendung des Stammes *Lactobacillus plantarum* P 1462 zur Herstellung von fermentierten Fischen und Original-Meeresprodukten und deren verarbeiteten Nebenprodukten.

4. Verwendung des Stammes *Lactobacillus plantarum* P 1462 zur Herstellung von fermentierten getrockneten Fischen und Original-Meeresprodukten und deren verarbeiteten Nebenprodukten.

## Revendications

1. Souche *Lactobacillus plantarum* de bactéries d'acide lactique, déposée à la collection de cultures de micro-organismes lettone (Latvian Culture Collection of Microorganisms) sous le numéro P 1462.

2. Produits, contenant la souche *Lactobacillus plantarum* P 1462 selon la revendication 1.

3. Utilisation de la souche *Lactobacillus plantarum* P 1462 pour la production de produits de poisson fermenté et d'origine marine et de leurs sous-produits de traitement.

4. Utilisation de la souche *Lactobacillus plantarum* P 1462 pour la production de produits de poisson déshydraté et d'origine marine et de leurs sous-produits de traitement.
